# EUROPEAN PATENT SPECIFICATION

(11) **EP 4 029 541 B1**
(45) Date of publication and mention of the grant of the patent: **03.06.2026**
(21) Application number: 22151455.7
(22) Date of filing: 13.01.2022
(51) Int. Cl.: A61M 1/16, A61M 1/34, A61M 1/36, G01F 1/684, G01F 1/69

(54) **MEASURING APPARATUS AND ARTIFICIAL DIURESIS DEVICE**
MESSVORRICHTUNG UND KÜNSTLICHE DIURESEVORRICHTUNG
APPAREIL DE MESURE ET DISPOSITIF DE DIURÈSE ARTIFICIEL

(30) Priority: 13.01.2021 IT 202100000521
(43) Date of publication of application: 20.07.2022
(73) Proprietor: Medica S.p.A., 41036 Medolla (IT)
(72) Inventor: FECONDINI, Luciano, 41036 Medolla (MO) (IT); RONCO, Claudio, 41036 Medolla (MO) (IT)
(74) Representative: Studio Torta S.p.A.

(56) References cited:
- EP-A1- 3 566 729
- WO-A1-2014/111908
- US-A1- 2015 144 543

## Description

### CROSS-REFERENCE TO RELATED APPLICATIONS

This Patent Application claims priority from Italian Patent Application No. 102021000000521 filed on January 13, 2021.

### TECHNICAL FIELD

The present invention concerns a measuring apparatus and an artificial diuresis device.

### PRIOR ART

In various clinical situations elimination of the excess fluid accumulated in the organism due to various pathologies (heart failure, kidney failure, edematous disorders) together with the urine (physiological diuresis) is inadequate or insufficient. In these conditions the fluid overload causes serious clinical complications, increase in hospitalization and mortality. In many of these cases, diuretic-based pharmacological solutions are used to obtain an increase in renal urine excretion (forced diuresis). Nevertheless, in a significant percentage of patients the pharmacological approach does not achieve the desired outcome and the medical condition of fluid overload persists in the patient, with negative clinical and social outcomes.

The use of techniques for removal of the excess fluid by means of extracorporeal ultrafiltration is known, however the equipment is cumbersome and suitable for use only in highly specialized settings due to the complexity of use and potential risks for the patient in the case of incorrect use. Said risks are connected with the high volume of blood present in the extracorporeal circuit and the flow necessary to treat the blood with traditional filters for adults: inappropriate use can cause loss of the blood which cannot be restored to the patient, and can result in serious drops in body blood pressure.

Therefore, the ultrafiltration equipment of known type is not commonly used to support other therapies or in departments for different pathologies due both to its cumbersomeness and the need for the presence of specialist staff. In these situations, therefore, pharmacological treatments are frequently used to support other therapies; however, pharmacological treatment is not as effective as the use of ultrafiltration equipment.

By ultrafiltration we mean an established and well-known process for the production of plasma water, but not cells or colloids, from blood containing electrolytes and cristalloids by means of separation obtained through a membrane.

### DESCRIPTION OF THE INVENTION

The object of the present invention is, therefore, to provide a measuring apparatus and an artificial diuresis device that overcome the drawbacks described above.

The object of the present invention is to provide a simple, portable and safe device that makes the technique of ultrafiltration feasible in any setting, either in hospital or at home, facilitating any self-administration of the therapy by the patient.

According to the present invention a measuring apparatus and an artificial diuresis device are provided according to the attached claims.

### BRIEF DESCRIPTION OF THE DRAWINGS

For a better understanding of the invention embodiments are described, purely by way of example, in which:
- figure 1 is a schematic view with some parts removed for clarity of a preferred embodiment of an artificial diuresis device according to the present invention;
- figure 2 illustrates a detail of the device of figure 1;
- figure 3 is a schematic view of a detail of the device of figure 1 in an operating configuration;
- figure 4 illustrates a detail of figure 3;
- figure 5 is the section according to the line V-V of figure 4;
- figure 6 is the section according to the line VI-VI of figure 4;
- figure 7 is the section according to the line VII-VII of figure 4; and
- figure 8 is a schematic view of a detail of the device of figure 1.

### PREFERRED EMBODIMENTS OF THE INVENTION

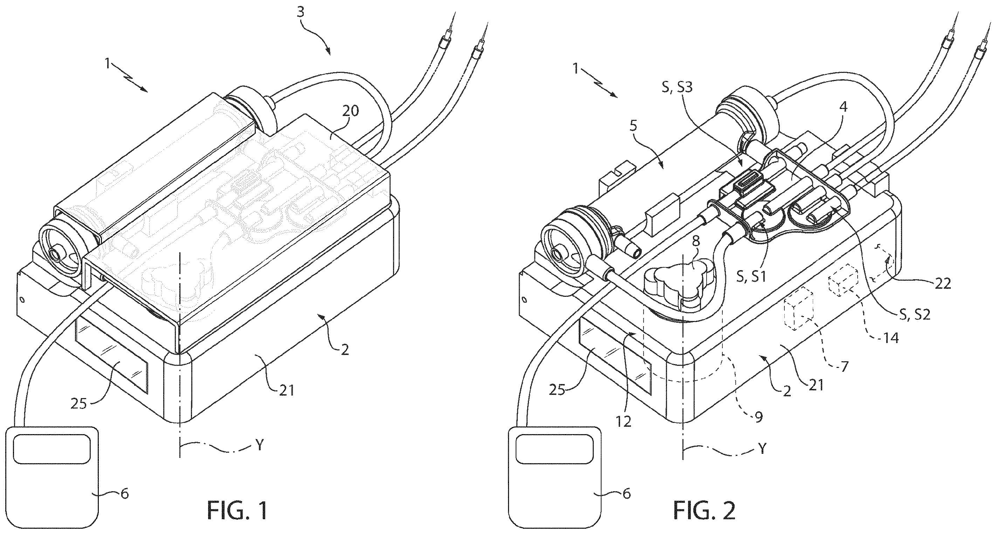

In figure 1 the number 1 indicates overall an artificial diuresis device according to the present invention. Advantageously, the artificial diuresis device 1 is portable, namely it can be worn by a patient H allowing movements (such as walking, sitting and the like). In other words, the artificial diuresis device 1 has reduced dimensions and weight so that it can be lifted and moved easily by any person.

The device 1 is configured to work with a volume of extracorporeal blood of less than 40 ml, preferably 30 ml.

The device 1, being portable, having reduced dimensions and working with a volume of extracorporeal blood of less than 40 ml, intrinsically reduces patient risk in the event of blood loss.

The artificial diuresis device 1 comprises a reusable machine 2 and a disposable unit 3.

As illustrated schematically in figure 2, the machine 2 comprises a box-like body 21 having an inner cavity 22; in this way, advantageously, the part of the most costly and cumbersome components of the device 1 are enclosed within the cavity 22 of the machine 2 (and are not accessible) so that they can be used several times.

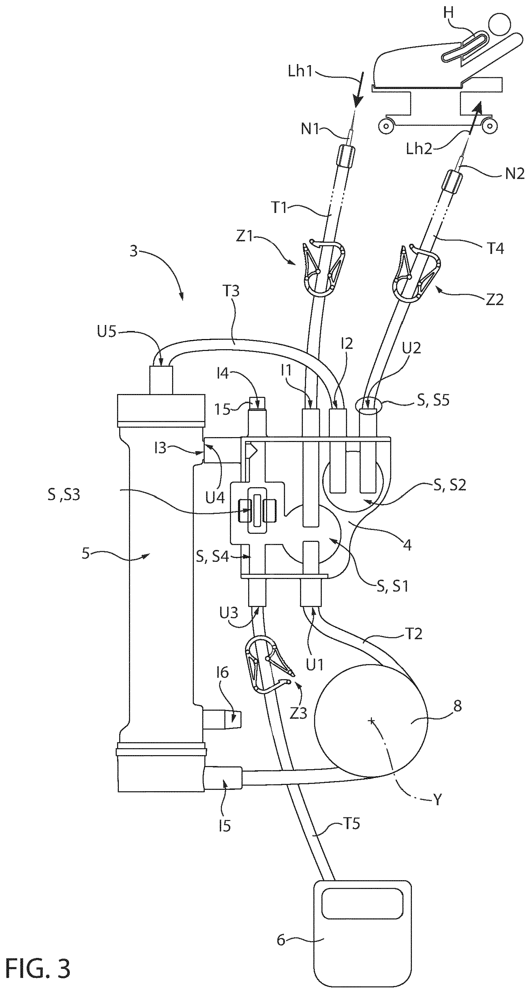

As illustrated in further detail in figure 3, the disposable unit 3 can be used one single time and comprises components that come into contact, in use, with the organic liquids Lh of the patient H. At the end of each treatment the disposable unit 3 is disposed of in compliance with the law.

As illustrated schematically in figure 3, the disposable unit is connected, in use, to the patient H by means of:
- a vascular access device N1 (of known type and illustrated schematically) to extract organic liquid Lh1, which is fed into the diposable unit 3;
- a vascular access device N2 (of known type and illustrated schematically) for reinfusion of the treated organic liquid Lh2 into the patient H.

For example, the vascular access devices N1 and N2 are needles and/or catheters or similar. Each vascular access device N1, N2 is connected, in use, to the disposable unit 3 as will be illustrated in greater detail below.

Advantageously, as will be illustrated in greater detail below, the disposable unit 3 is completely filled, before use, with treatment liquid Lt. The treatment liquid Lt can be saline solution, if necessary mixed with other substances such as heparin, for example.

According to the example illustrated in figure 1, the device 1 further comprises a cover 20 having substantially the form of a cup-shaped body with concavity facing, when coupled, the body 21 of the machine 2 coupled with the machine 2. In use the cover 20 covers and screens, at least partly, the disposable unit 3.

According to the example illustrated, the disposable unit 3 comprises: an operating box 4; a plurality of flexible tubes T for the flow of the organic liquids Lh; a filtering unit 5; and a collecting bag 6.

Advantageously, the device 1 comprises a plurality of measuring apparatuses S, as will be illustrated in further detail below, in order to verify correct execution of the treatment.

The device 1 further comprises a control unit 7, which is housed inside the inner cavity 22 and with which each measuring apparatus S exchanges signals, in use.

Advantageously, the body 21 of the machine 2 and the box 4 are configured to couple with each other, for example by means of shape and/or interference couplings (of known type and not illustrated), in a predefined position, as will be illustrated in greater detail below.

Advantageously, the box 4 is made in one single piece. Advantageously the box 4 is made by molding of polymeric material. In this way, the box 4 is lightweight and easy to wear.

Furthermore, the box 4 is optimized so as to reduce the overall dimensions and thus facilitate portability.

Advantageously, the box 4 is configured so as to minimize the volume of blood present in the extracorporeal circuit of the disposable unit 3.

Preferably, the box 4 is configured to limit the volume of blood present in the extracorporeal circuit to below 40 ml, preferably approximately 30 ml.

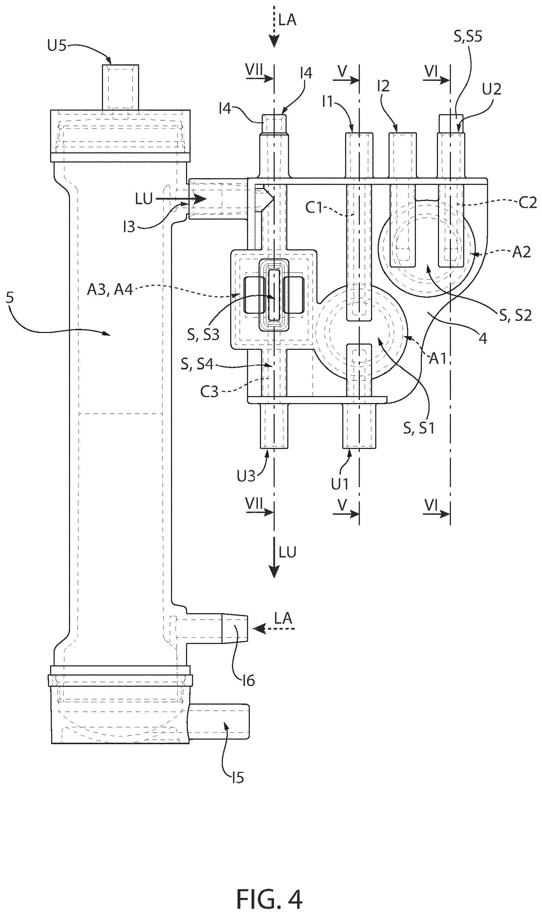

As illustrated in figures 3 and 4, the box 4 has:
- an inlet I1 for organic liquid Lh1 to be treated and coming from the patient H;
- an outlet U1 for sending the organic liquid Lh1 to be treated to the filtering unit 5;
- an inlet channel C1 that connects the inlet I1 to the outlet U1;
- an inlet I2 for the treated organic liquid Lh2 coming from the filtering unit 5;
- an outlet U2 for sending the treated organic liquid Lh2 to the patient H; and,
- an outlet channel C2 that connects the inlet I2 to the outlet U2.

Advantageously, the box 4 has an interaction area A1 configured to interface, in use, with the machine 2. The interaction area A1 is arranged along the inlet channel C1. According to the example illustrated, the interaction area A1 has a substantially cylindrical shape. Without loss of generality, the interaction area A1 can have different shapes and dimensions from those illustrated.

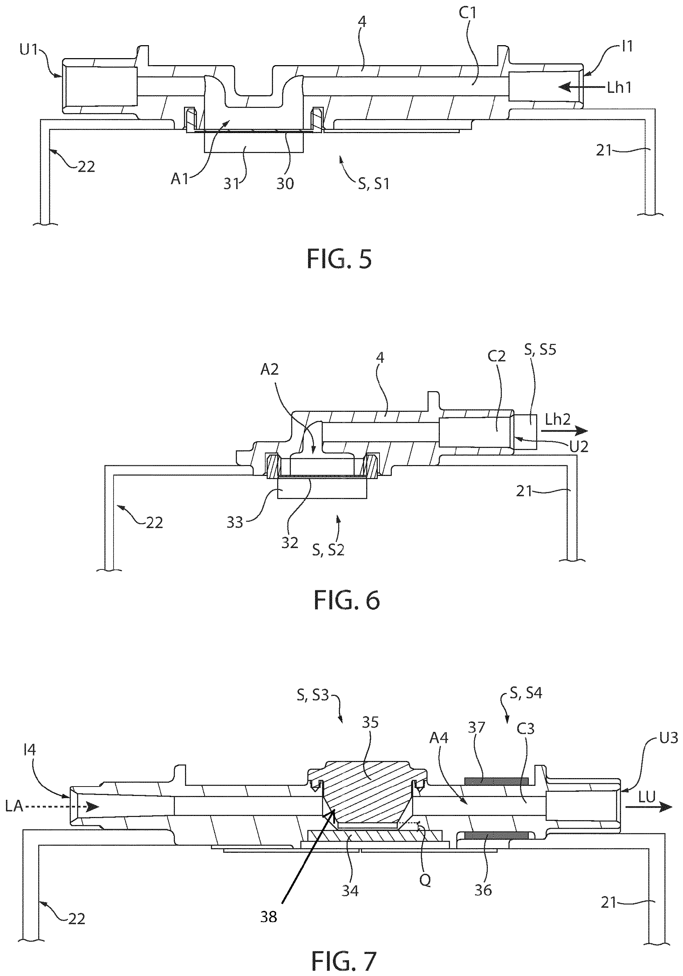

The device 1 comprises a measuring apparatus S1 (schematized in figure 5) which is configured to detect the pressure of the organic liquid Lh1 to be treated in the interaction area A1.

Preferably, the box 4 comprises a membrane 30 which laterally delimits a respective portion of the interaction area A1. The machine 2 comprises, in turn, a pressure transducer 31 which detects, in use, variations in the pressure of the organic liquid Lh1 to be treated inside the interference area A1 according to the deformation of the membrane 31. The pressure transducer 31 is housed inside the cavity 22 of the machine 2 and exchanges signals (in a known manner not illustrated) with the control unit 7.

Advantageously, the box 4 has an interaction area A2 configured to interface, in use, with the machine 2. The interaction area A2 is arranged along the outlet channel C2. According to the example illustrated, the interaction area A2 has a substantially cylindrical shape. Without loss of generality, the interaction area A2 can have shapes and dimensions different from those illustrated.

The device 1 comprises a measuring apparatus S2 (schematized in figure 5) which is configured to detect the pressure of the treated organic liquid Lh2 flowing out of the filtering unit 5 and in the area of the interaction area A2. Preferably, the box 4 comprises a membrane 32 that laterally delimits a respective portion of the interaction area A2. The machine 2 comprises, in turn, a pressure transducer 33 which detects, in use, variations in the pressure of the treated organic liquid Lh2 inside the interference area A2 according to the deformation of the membrane 32. The pressure transducer 33 is housed inside the cavity 22 of the machine 2 and exchanges signals (in a known manner not illustrated) with the control unit 7.

Advantageously, the box 4 furthermore has:
- an inlet I3 for the ultra-filtered liquid LU; and
- an outlet U3 for sending the ultra-filtered liquid LU to the collecting bag 6; and,
- an ultra-filtered channel C3 that connects the inlet I3 to the outlet U3.

Advantageously, as will be illustrated in greater detail below, the box 4 furthermore has an inlet I4 for introducing auxiliary liquid LA into the ultra-filtered channel C3. The auxiliary liquid LA can correspond to the treatment liquid Lt or be of different type.

Advantageously, the box 4 has an interaction area A3 configured to interface, in use, with the machine 2. In particular, the machine 2 comprises, in turn, a measuring apparatus S3 for detecting the flow of the ultra-filtered liquid LU in the interaction area A3.

The measuring apparatus S3 comprises a flowmeter 34.

According to the invention as claimed, the measuring apparatus S3 comprises a flow adjustment element 35 which cooperates with the flowmeter 34. As claimed and as illustrated in figure 7, the adjustment element 35 is a cartridge which has a shaped profile 38 facing in use the inside of the ultra-filtered channel C3 and in contact with the ultra-filtered liquid LU.

The form of the shaped profile 38 is determined according to the desired flow rate value of the ultra-filtered liquid LU in the interaction area A3, in particular in the area of the flowmeter 34.

Advantageously the cartridge 35 is fixed to the box 4 in the interaction area A3, so as to delimit a respective section Q of the ultra-filtered channel C3.

Advantageously, the flowmeter 34 cooperates with the cartridge 35 so as to delimit a respective section Q of the ultra-filtered channel C3.

The flowmeter 34 and the cartridge 35 delimit laterally, at least partially, the same passage section Q as the ultra-filtered channel C3.

According to a variation not illustrated, the adjustment element 35, namely the cartridge 35, and the flowmeter 34 are in two distinct and separate positions along the channel C3. For example, the flowmeter 34 and the adjustment element 35 do not face each other.

Advantageously, the cartridge 35 is chosen from within a group of cartridges 35 different from one another in terms of the shape and dimension of the shaped profile 38 intended to be in contact, in use, with the ultra-filtered liquid LU.

Advantageously the form and dimension of the shaped profile 38 of a cartridge 35 obtain respective different flow rates of the ultra-filtered liquid LU which flows through the passage section Q.

According to a variation not illustrated, the adjustment element 35 is made in one single piece with the box 4. In other words, the adjustment element 35 is substantially obtained with a corresponding appropriately shaped portion of the box 4.

For example the flowmeter 34 is produced in principle according to the teachings described in the patent application EP 3 566 729 A1 or in the patent application EP 2 946 179 A1, the teachings of which are considered included herein.

Advantageously but without limitation, the flowmeter 34 comprises an electric/electronic measuring circuit 41, which is provided with at least one thermo-resistive electric component 42. The thermo-resistive electric component 42 can expediently comprise, for example, a thermal sensor, preferably a thermal anemometer, the thermo-resistive (sensitive) electric component of which is used both as a dissipative sensor and as a temperature sensor. It is understood that the thermo-resistive electric component 42 is used for temperature compensation of the fluid, thus making the flow rate independent of the fluid temperature.

Preferably, the thermo-resistive electric component 42 can comprise a PTC (Positive Temperature Coefficient) resistor or any similar thermistor electric/electronic component.

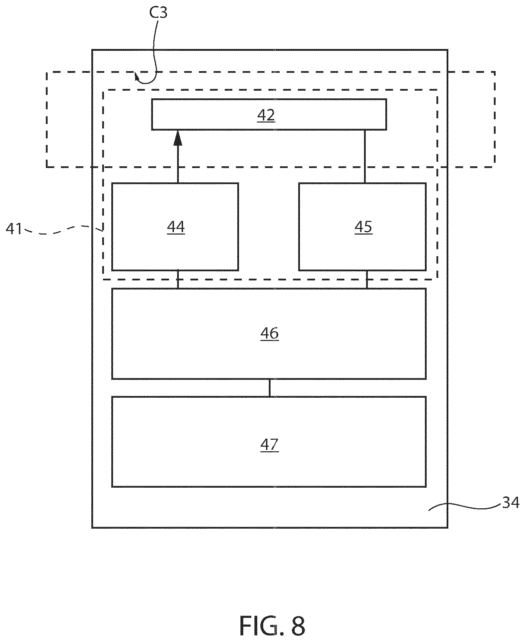

The thermo-resistive electric component 42 can be associated/coupled/arranged with/in the ultra-filtered channel C3 (schematized by a broken line in figure 8) so that it can transmit and/or receive heat, directly or indirectly, to/from the liquid that crosses the ultra-filtered channel C3.

In this way, the thermo-resistive electric component 42 is expediently lapped by the ultra-filtered liquid LU (coming into contact with the ultra-filtered liquid LU) and varies its resistance according to the temperature and flow rate of the ultra-filtered liquid LU.

According to a variation not illustrated, the ultra-filtered channel C3 can be at least partially provided through the flowmeter 34.

The measuring circuit 41 can further comprise a voltage measuring device 45, which is electrically coupled to the thermo-resistive component 42, for example at the (terminal) ends thereof. The voltage measuring device 45 is configured so as to measure the electric voltages VL and VH at the ends of the resistive component 42.

The flowmeter 34 can further comprise a calculation device 46 configured so as to implement the calculation operations to determine the liquid flow rate.

The calculation device 46 can be programmed/configured so as to:
- receive from the voltage measuring device 45 the voltages VL and VH of the thermo-resistive component 42 measured according to predefined methods;
- calculate respective resistive values R indicative of the electrical resistance of the thermo-resistive component 42.

Advantageously, the flowmeter 34 is an integrated electromechanical microsystem in miniaturized form, generally known as MEMS (Micro Electro-Mechanical System). In other words, advantageously, the flowmeter 34 is miniaturized.

The flowmeter 34 is disposable, namely it can be used one single time.

The flowmeter 34 is configured to exchange signals relative to the resistive values R detected with the control unit 7.

Advantageously, the box 4 has an interaction area A4 configured to interface, in use, with the machine 2. In particular, the machine 2 comprises, in turn, a measuring apparatus S4 for measuring blood traces to detect any blood losses in the ultra-filtered liquid LU in the interaction area A4.

For example, the measuring apparatus S4 comprises an optical sensor 36, for example a sensor of the type generally known as BLD. The measuring apparatus S4 further comprises a reflecting element 37 which faces the optical sensor 36 so that, in use, when the ultra-filtered liquid LU passes through the interaction area A4, it flows between the optical sensor 36 and the reflecting element 37. For example, the reflecting element 37 is a mirror surface that laterally delimits the ultra-filtered channel C3.

Advantageously, the reflecting element 37 is configured to increase the contrast so as to optimize the detection of any blood traces.

Without loss of generality and by way of example, according to the example illustrated in figure 7, the interaction areas A3 and A4 are distinct areas.

According to a variation not illustrated, the interaction areas A3 and A4 coincide and are overlapped, namely detection of the flow and the presence of blood occur in one single area.

According to a variation not illustrated, the flowmeter 34 and the optical sensor 36 are inserted inside a cartridge 35 configured to be applied, by means of a shape and/or interference coupling, to the box 4 in the interaction area A3/A4. The differential flowmeter 34 and the optical sensor 36 face the inside of the ultra-filtered channel C3.

According to a variation not forming part of the invention as claimed, the measuring apparatus S4 of the type described above can be applied on different devices from those for artificial diuresis.

Advantageously, the device 1 comprises a measuring apparatus S5 for detecting bubbles in the treated organic liquid Lh2 before it is sent to the patient H. For example, the measuring apparatus S5 is applied in the area of the outlet U2.

Advantageously, the device 1 further comprises, for each vascular access device N1, N2 a measuring apparatus S6 for detecting correct insertion of the latter during the treatment. Advantageously, each measuring apparatus S, S1, S2, S3, S4, S5 and S6 exchanges signals, in use, with the control unit 7.

According to the example illustrated, the filtering unit 5 is a filter. According to a variation not illustrated, the filtering unit can comprise a plurality of filters installed in parallel or in series. The filtering unit 5 is of known type and is schematically illustrated.

By way of non-exhaustive example, the filtering unit 5 is a hollow capillary fiber hemofilter with cutoff lower than 55 kDa so as to avoid albumin losses in the ultra-filtered liquid LU. The hollow capillary membrane of the filtering unit 5 can be made of polysulfone (Medisulfone^{®}, produced by Medica S.p.A.) or, alternatively, polyethersulfone/polyvinylpyrrolidone (generally known as PUREMA^{®} H membrane produced by 3M^{®}).

Advantageously, the filtering unit 5 guarantees a flow of ultra-filtered liquid LU equal to at least 5% of the flow of the pump 12. For example, the flow of the ultra-filtered liquid LU is between 1 ml/min and 4 ml/min.

According to a variation not illustrated, the filtering unit 5 is not a hemofilter but a hollow capillary fiber plasmafilter, with membrane in PES/PVP produced by Medica SpA (Versatile-PES^{®}) which performs plasma filtration, namely separation of the blood cell components (which remain in the lumen of the fibers) from the plasma. Said filter has a cutoff of approximately 1000 kDa and advantageously allows slow continuous plasma filtering.

In a known manner, the filtering unit 5 has:
- an inlet I5 for the incoming organic liquid Lh1;
- an outlet U4 for the ultra-filtered liquid LU;
- an outlet U5 for the treated organic liquid Lh2.

The filtering unit 5 further comprises an inlet I6 for auxiliary liquid LA which can be inserted by manual pumping means preloaded for any backflush.

As illustrated in figure 3, the outlet U4 is in fluidic communication with the inlet I3 of the box 4.

As illustrated in figures 1 and 2, the artificial diuresis device 1 further comprises a pump 12, in particular a peristaltic pump, for circulation of the organic liquid Lh through the disposable unit 3. Advantageously, the pump 12 comprises a head 8, which protrudes outside the body 21 of the machine 2, and an operation unit 9, which is inserted inside the cavity 22 of the machine 2 and, in use, rotates the head 8 around an axis of rotation Y. The head 8 of the peristaltic pump is configured to interact with the disposable unit 3, as will be illustrated in greater detail below, so as to cause circulation of the organic liquid Lh. The peristaltic pump 12 is of known type and schematically illustrated.

The pump 12 is configured to obtain a pumping of 20-40 ml/minute.

In other words, the flow rate of the pump 12 is certainly lower than that of the pumps commonly used in the fixed machines of known type for hemofiltration which is in the order of magnitude of 200 ml/minute.

The disposable unit 3 further comprises:
- an inlet tube T1 which is fluidically connected to the inlet I1 and is configured to connect, in use, to the vascular access device N1 for drawing organic liquid Lh1 from the patient H;
- a tube T2 that fluidically connects the outlet U1 of the box 4 to the inlet I5 of the filtering unit 5;
- a tube T3 that fluidically connects the outlet U5 of the filtering unit 5 to the inlet I2 of the box 4;
- a tube T4 which is fluidically connected to the outlet U2 of the box 4 and is configured to connect, in use, to the vascular access device N2 for the reinfusion of treated organic liquid Lh2 into the patient H; and
- a tube T5 that connects the outlet U3 of the box 4 to the collecting bag 6.

The tubes T1, T2, T3, T4 and T5 indicated above are of known type and schematically illustrated. In particular, they are made of flexible PVC and are disposable.

Advantageously, the device 1 comprises one or more electric contacts (of known type and not illustrated) between the box 4 and the machine 2 for the passage of signals from one or more sensitive elements S, S1, S2, S3, S4 to the control unit 7.

Advantageously, the device 1 is powered by a battery 14. In this way, advantageously, the device 1 does not have to be connected to a fixed electric power supply network, allowing a patient H to move during the treatment.

The device 1 further comprises a plurality of clamps Z of known type and schematically illustrated, each of which is configured to close the area of passage of a respective tube T so as to stop the passage of fluid. In particular, the device 1 comprises:
- a clamp Z1 applied to the tube T1;
- a clamp Z2 applied to the tube T5; and
- a clamp Z3 applied to the tube T1.

Advantageously, the device 1 further comprises a needle free valve 15 applied to close the inlet I4 of the box 4. The valve 15 is of known type and is schematically illustrated. Advantageously, an operator can inject liquid, by means of a syringe, into the ultra-filtered channel C3 through the valve 15.

Advantageously, before use, the disposable unit 3 is completely filled with saline solution. This guarantees the absence of air inside the disposable unit 3 at the time of connection with the vascular access devices N1 and N2 and it is possible to immediately use the device 1 without carrying out a series of operations, generally known as priming operations, which necessarily have to be carried out on board fixed hemofiltration machines.

In other words, before the first use the following are completely filled with saline solution: the tube T1, the inlet channel C1, the tube T2, the filtering unit 5, the tube T3, the outlet channel C2, the tube T4, the ultra-filtered channel C3 and the tube T5.

Advantageously, the device 1 of the type described above is portable, therefore it can be used by a patient H in any place, at the same time allowing him/her to move.

Advantageously, the device 1 of the type described above does not require the intervention of medical staff specialized in hemofiltration treatments in order to be applied to the patient.

Therefore the device 1 of the type described above can be used also in any type of department and by medical staff not specialized in hemofiltration.

According to the example illustrated, the machine 2 further comprises a display 25 which dialogues with the control unit 7 and is configured to exchange data with the user. For example, the display can be a graphic interface applied to the outside of the box-like body 21 of the machine 2. Alternatively, the display can be a graphic interface of a remote device such as, for example, the screen of a computer, tablet, smartphone and the like.

In use, the machine 2 and the disposable unit 3 are pre-arranged.

Advantageously, the disposable unit 3 is hermetically sealed inside sterile packaging and is pre-filled with saline solution so as to guarantee the use thereof without the need to carry out a circuit preparation and filling phase, as with traditional devices.

The disposable unit 3, once removed from its packaging, is applied to the box-like body 21 of the machine 2. In particular, the box 4 is coupled to the body 21 by means of the shape and/or interference couplings provided so as to guarantee that the disposable unit 3 assumes a predefined position during use.

Therefore, the tubes T are positioned in a predefined manner.

In particular, the tube T2 is partially wound around the head 8 of the pump 12. The cartridge 35 is inserted in its housing if not already present. The collecting bag 6 is connected to the tube T5 if not already present.

Simultaneously or subsequently to pre-arrangement of the disposable unit 3 on the machine 2, the vascular access devices N1 and N2 are applied to the patient H according to hemofiltration treatment methods known and not illustrated.

The vascular access device N1 for drawing organic liquid Lh1 from the patient H is then connected to the inlet tube T1; analogously, the vascular access device N2 for the reinfusion of treated organic liquid Lh2 is connected to the outlet tube T2.

When the ignition signal of the device 1 is given, for example via the display 25, the pump 12 is operated, causing the organic liquid Lh1 coming from the tube T1 to flow, pushing it into the filtering unit 5.

The organic liquid Lh1 taken from the patient H enters the channel C1 of the disposable unit 3 and passes through the interaction area A1. In the interaction area A1 the pressure measuring apparatus S1 detects the inlet pressure P1 of the incoming organic liquid Lh1. Advantageously, the inlet pressure value P1 serves to determine if there are obstructions or if the vascular access device N1 has become detached.

Once the organic liquid Lh1 has flowed out of the inlet channel C1, it passes through the tube T2 and is pushed by the pump 12 into the filtering unit 5.

The filtering unit 5 separates, in a known manner, the ultra-filtered liquid LU from the treated organic liquid Lh2.

At the outlet of the filtering unit 5, the treated organic liquid Lh2 is pushed into the outlet channel C2 and passes through the interaction area A2.

In the interaction area A2 the pressure measuring apparatus S2 detects the outlet pressure P2 of the treated organic liquid Lh2.

Advantageously, the outlet pressure value P2 serves to determine if there are any obstructions or if the vascular access device N2 has become detached.

As will be illustrated in greater detail below, in the case of bubbles being detected in the treated organic liquid Lh2, the measuring apparatus S5 exchanges signals with the control unit 7 to stop operation of the device 1.

The ultra-filtered liquid LU is pushed as it flows out of the filtering unit 5 through the outlet U4 into the ultra-filtered channel C3.

In the ultra-filtered channel C3, the ultra-filtered liquid LU passes through the interaction areas A3 and A4. In the interaction areas A3 and A4 the flow rate is detected by means of the measuring apparatus S3 for detecting the flow and/or the presence of any blood traces by means of the blood measuring apparatus S4. At the outlet of the ultra-filtered channel C3, the ultra-filtered liquid LU enters the collecting bag 6.

If, during the treatment, the control unit 7 detects abnormal functioning based on the signals detected by one or more of the measuring apparatuses S; S1, S2, S3, S4, S5 the control unit adjusts, in particular it slows down or stops, the operation unit 9 of the pump 12. In this way, advantageously, the treatment is stopped in a relatively short time.

Advantageously, the control unit 7 has a software to adjust the operation unit 9 according to the type of treatment to be carried out.

The display 25 is configured to emit acoustic or visual alarms to draw the attention of the healthcare operators.

In order to carry out washing operations on the filtering unit 5, the clamp Z3 is tightened so as to close the passage section of the tube T5. An operator can then inject treatment liquid Lt through the needle free valve 15 and inside the ultra-filtering channel C3 so as to obtain back-washing of the filtering unit 5. Additionally or alternatively, an operator with pre-loaded manual pumping means can introduce auxiliary liquid LA through the inlet I6 for any backflush.

Advantageously, the device 1 of the type described above has reduced dimensions and is easy to apply.

Advantageously, given the reduced flow rate of the organic liquid Lh through the device 1, it is possible to use the device 1 to carry out the treatments for longer periods of time than those carried out with traditional apparatuses.

Given the reduced quantity of organic liquid Lh in circulation through the device 1, stopping of the device 1 does not harm the patient.

Advantageously, the device 1 of the type described above allows artificial diuresis to be obtained which is:
- continuous (the device 1 can carry out treatments lasting several hours, for example 24 hours or for fractions of a day, or a few hours);
- slow and progressive;
- adjustable and safe; and
- manageable at the patient's bed or even at home.

Advantageously, the device 1 of the type described above is simple to apply, thus allowing ultrafiltration processes to be performed also in situations where up to now these types of treatment were not applicable or could be obtained only by means of pharmaceutical treatment.

Purely by way of example, the device 1 can be used to carry out the following treatments:
1. Acute heart decompensation: slow progressive removal of fluid adjustable to a variable speed similar to physiological diuresis;
2. Critical condition in intensive care: management of fluid balance in the case of oliguria or excessive need for infusion, independently of dialysis therapy;
3. Recent kidney transplant: management of fluid balance in the case of slow graft function;
4. Patients in ECMO: management of fluid balance after or during extracorporeal oxygenation;
5. Decompensated nephrotic syndrome and anasarca: management of fluid overload;
6. Decompensating cirrhosis: management of anasarcatic and ascitic state;
7. Chronic heart decompensation: management of fluid overload in initial stages of decompensation (rescue therapy) or management of prevention of re-hospitalizations with periodic treatments (elective therapy);
8. Patient with initial kidney failure and need for fluid rebalance;
9. Hypoalbuminemia and generalized edematous states: reduction of fluid overload;
10. Pediatric patients requiring fluid balance optimization: the miniaturized nature of the device allows use in young patients right down to neonatal level;
11. Treatment of fluid overload in the interdialytic period in patients in thrice-weekly hemodialysis;
12. Periodic treatment of hyperhydration in patients in peritoneal dialysis with insufficient transperitoneal fluid removal;
13. Therapy in protected settings: for example care homes and limited care centers;
14. Home therapy: home management of chronic edema.

## Claims

1. A measuring apparatus (S3) for detecting the flow of a liquid (LU) through a channel (C3) of a portable artificial diuresis device (1), which is configured to work with a volume of extracorporeal blood of less than 40 ml; wherein said measuring apparatus (S3) comprises a flowmeter (34) which laterally delimits a respective portion of said channel (C3); said measuring apparatus (S3) being disposable; said measuring apparatus (S3) comprising an adjustment element (35) which laterally delimits, at least partly, said portion of the channel (C3) in which the liquid (LU) flows; said adjustment element (35) is a cartridge having a shaped profile (38) facing, in use, the inside of said channel (C3) and in contact with the liquid (LU); wherein, the form of the shaped profile (38) is determined according to the desired flow rate value of the liquid (LU) in the area of the flowmeter (34).

2. The measuring apparatus according to claim 1, wherein said flowmeter (34) comprises, in turn, an electric/electronic measuring circuit (41) configured to detect, in use, the flow of the liquid (LU), in particular the flow rate of the liquid (LU), independently of the temperature of the liquid (LU).

3. The measuring apparatus according to claim 2, wherein the electric/electronic measuring circuit (41) comprises a thermo-resistive electric component (42) which acts, in use, as a dissipation element and/or as a temperature sensor.

4. The measuring apparatus according to claim 3, wherein said thermo-resistive electric component (42) comprises a thermal sensor, in particular with anemometer.

5. The measuring apparatus according to claim 3 or 4, wherein the thermo-resistive electric component (42) comprises a thermistor electric/electronic component, in particular a PTC (Positive Temperature Coefficient) thermistor.

6. The measuring apparatus according to any one of the claims from 3 to 5, wherein said thermo-resistive electric component (42) can be associated with said channel (C3) so that, in use, there is transmission of heat between the liquid (LU) that passes through said channel (C3) and the thermo-resistive electric component (42); wherein said thermo-resistive electric component (42) varies, in use, its electric resistance according to the temperature and flow rate of the liquid (LU).

7. The measuring apparatus according to claim 6, wherein the thermo-resistive electric component (42) is lapped, in use, directly by the liquid (LU).

8. The measuring apparatus according to any one of the claims from 3 to 7, wherein said measuring circuit (41) further comprises a voltage measuring device (45), which is electrically connected to said thermo-resistive component (42); wherein the voltage measuring device (45) measures, in use, the electric voltage of the resistive component (42).

9. The measuring apparatus according to any one of the claims from 3 to 8, wherein said measuring circuit (41) further comprises a calculation device (46) which implements, in use, the calculation operations to determine the flow rate of the liquid.

10. The measuring apparatus according to claim 9, wherein the calculation device (46) is configured to:
- receive from said measuring device (45) the electric voltages of the thermo-resistive component (42);
- calculate respective resistive values (R) indicative of the electric resistance of the thermo-resistive component (42).

11. The measuring apparatus according to any one of the preceding claims, wherein said flowmeter (34) is an integrated electromechanical microsystem in miniaturized form, generally known as MEMS (Micro Electro-Mechanical System); in particular, said flowmeter (34) is miniaturized.

12. The measuring apparatus according to any one of the preceding claims, wherein the adjustment element (35) and the flowmeter (34) laterally delimit the same predefined passage section (Q) of the channel (C3); the adjustment element (35) being configured to obtain a predefined flow rate of the liquid (LU) that flows in contact with the flowmeter (34).

13. The measuring apparatus according to any one of the preceding claims and comprising an optical sensor (36); in particular said measuring apparatus further comprises a reflecting element (37) which faces the optical sensor (36) so that, in use, the liquid (LU) flows between the optical sensor (36) and the reflecting element (37).

14. An artificial diuresis device comprising a measuring apparatus (S3) according to any one of the preceding claims.

## Patentansprüche

1. Messgerät (S3) zum Erfassen des Durchflusses einer Flüssigkeit (LU) durch einen Kanal (C3) einer tragbaren künstlichen Diuresevorrichtung (1), die konfiguriert ist, um mit einem Volumen von extrakorporalem Blut von weniger als 40 ml zu arbeiten; wobei das Messgerät (S3) einen Durchflussmesser (34) umfasst, der einen jeweiligen Abschnitt des Kanals (C3) seitlich begrenzt; wobei das Messgerät (S3) wegwerfbar ist; wobei das Messgerät (S3) ein Einstellelement (35) umfasst, das den Abschnitt des Kanals (C3), in dem die Flüssigkeit (LU) strömt, zumindest teilweise seitlich begrenzt; wobei das Einstellelement (35) eine Kartusche mit einem geformten Profil (38) ist, das im Gebrauch dem Inneren des Kanals (C3) zugewandt ist und mit der Flüssigkeit (LU) in Kontakt steht; wobei die Form des geformten Profils (38) gemäß dem gewünschten Durchflussratenwert der Flüssigkeit (LU) im Bereich des Durchflussmessers (34) bestimmt wird.

2. Messgerät nach Anspruch 1, wobei der Durchflussmesser (34) wiederum eine elektrische/elektronische Messschaltung (41) umfasst, die konfiguriert ist, um im Gebrauch den Durchfluss der Flüssigkeit (LU), insbesondere die Durchflussrate der Flüssigkeit (LU), unabhängig von der Temperatur der Flüssigkeit (LU) zu erfassen.

3. Messgerät nach Anspruch 2, wobei die elektrische/elektronische Messschaltung (41) eine thermoresistive, elektrische Komponente (42) umfasst, die im Gebrauch als Ableitungselement und/oder als Temperatursensor wirkt.

4. Messgerät nach Anspruch 3, wobei die thermoresistive, elektrische Komponente (42) einen thermischen Sensor, insbesondere mit Anemometer, umfasst.

5. Messgerät nach Anspruch 3 oder 4, wobei die thermoresistive, elektrische Komponente (42) eine elektrische/elektronische Thermistor-Komponente, insbesondere einen PTC (Positive Temperature Coefficient)-Thermistor, umfasst.

6. Messgerät nach einem der Ansprüche 3 bis 5, wobei die thermoresistive, elektrische Komponente (42) dem Kanal (C3) zugeordnet werden kann, so dass im Gebrauch eine Wärmeübertragung zwischen der Flüssigkeit (LU), die durch den Kanal (C3) strömt, und der thermoresistiven, elektrischen Komponente (42) stattfindet; wobei die thermoresistive, elektrische Komponente (42) im Gebrauch ihren elektrischen Widerstand entsprechend der Temperatur und Durchflussrate der Flüssigkeit (LU) variiert.

7. Messgerät nach Anspruch 6, wobei die thermoresistive, elektrische Komponente (42) im Gebrauch direkt von der Flüssigkeit (LU) umspült wird.

8. Messgerät nach einem der Ansprüche 3 bis 7, wobei die Messschaltung (41) ferner eine Spannungsmessvorrichtung (45) umfasst, die elektrisch mit der thermoresistiven Komponente (42) verbunden ist; wobei die Spannungsmessvorrichtung (45) im Gebrauch die elektrische Spannung der resistiven Komponente (42) misst.

9. Messgerät nach einem der Ansprüche 3 bis 8, wobei die Messschaltung (41) ferner eine Berechnungsvorrichtung (46) umfasst, die im Gebrauch die Berechnungsoperationen implementiert, um die Durchflussrate der Flüssigkeit zu bestimmen.

10. Messgerät nach Anspruch 9, wobei die Berechnungsvorrichtung (46) konfiguriert ist, um:
- von der Messvorrichtung (45) die elektrischen Spannungen der thermoresistiven Komponente (42) zu empfangen;
- jeweilige Widerstandswerte (R) zu berechnen, die den elektrischen Widerstand der thermoresistiven Komponente (42) angeben.

11. Messgerät nach einem der vorhergehenden Ansprüche, wobei der Durchflussmesser (34) ein integriertes elektromechanisches Mikrosystem in miniaturisierter Form ist, allgemein bekannt als MEMS (Micro Electro-Mechanical System); insbesondere ist der Durchflussmesser (34) miniaturisiert.

12. Messgerät nach einem der vorhergehenden Ansprüche, wobei das Einstellelement (35) und der Durchflussmesser (34) seitlich den gleichen vordefinierten Durchlassabschnitt (Q) des Kanals (C3) begrenzen; wobei das Einstellelement (35) konfiguriert ist, um eine vordefinierte Durchflussrate der Flüssigkeit (LU) zu erhalten, die in Kontakt mit dem Durchflussmesser (34) strömt.

13. Messgerät nach einem der vorhergehenden Ansprüche und umfassend einen optischen Sensor (36); insbesondere umfasst das Messgerät ferner ein reflektierendes Element (37), das dem optischen Sensor (36) zugewandt ist, so dass im Gebrauch die Flüssigkeit (LU) zwischen dem optischen Sensor (36) und dem reflektierenden Element (37) strömt.

14. Künstliche Diuresevorrichtung, umfassend ein Messgerät (S3) nach einem der vorhergehenden Ansprüche.

## Revendications

1. Appareil de mesure (S3) de détection de l'écoulement d'un liquide (LU) à travers un canal (C3) d'un dispositif portable (1) de diurèse artificielle, qui est configuré pour fonctionner avec un volume de sang extracorporel inférieur à 40 ml ; ledit appareil de mesure (S3) comprenant un débitmètre (34) qui délimite latéralement une portion respective dudit canal (C3) ; ledit appareil de mesure (S3) étant jetable ; ledit appareil de mesure (S3) comprenant un élément de réglage (35) qui délimite latéralement, au moins en partie, ladite portion du canal (C3) dans laquelle le liquide (LU) s'écoule ; ledit élément de réglage (35) étant une cartouche présentant un profil façonné (38) faisant face, lors de l'utilisation, à l'intérieur dudit canal (C3) et étant en contact avec le liquide (LU) ; la forme du profil façonné (38) étant déterminée selon la valeur de débit souhaitée du liquide (LU) dans la zone du débitmètre (34).

2. Appareil de mesure selon la revendication 1, ledit débitmètre (34) comprenant, à son tour, un circuit de mesure électrique/électronique (41) configuré pour détecter, lors de l'utilisation, le débit du liquide (LU), en particulier le débit du liquide (LU), indépendamment de la température du liquide (LU).

3. Appareil de mesure selon la revendication 2, le circuit de mesure électrique/électronique (41) comprenant un composant électrique thermo-résistif (42) qui agit, lors de l'utilisation, comme un élément de dissipation et/ou comme un capteur de température.

4. Appareil de mesure selon la revendication 3, le composant électrique thermo-résistif (42) comprenant un capteur thermique, en particulier un anémomètre.

5. Appareil de mesure selon la revendication 3 ou 4, le composant électrique thermo-résistif (42) comprenant un composant électrique/électronique de thermistance, en particulier une thermistance CTP (à coefficient de température positif).

6. Appareil de mesure selon l'une quelconque des revendications de 3 à 5, ledit composant électrique thermo-résistif (42) pouvant être associé audit canal (C3) de sorte que, lors de l'utilisation, il y a transmission de chaleur entre le liquide (LU) qui passe à travers ledit canal (C3) et le composant électrique thermo-résistif (42) ; ledit composant électrique thermo-résistif (42) faisant varier, lors de l'utilisation, sa résistance électrique selon la température et le débit du liquide (LU).

7. Appareil de mesure selon la revendication 6, le composant électrique thermo-résistif (42) étant recouvert, lors de l'utilisation, directement par le liquide (LU).

8. Appareil de mesure selon l'une quelconque des revendications de 3 à 7, ledit circuit de mesure (41) comprenant en outre un dispositif de mesure (45) de tension, qui est relié électriquement audit composant thermo-résistif (42) ; le dispositif de mesure (45) de tension mesurant, lors de l'utilisation, la tension électrique du composant résistif (42).

9. Appareil de mesure selon l'une quelconque des revendications de 3 à 8, ledit circuit de mesure (41) comprenant en outre un dispositif de calcul (46) qui met en œuvre, lors de l'utilisation, les opérations de calcul pour déterminer le débit du liquide.

10. Appareil de mesure selon la revendication 9, le dispositif de calcul (46) étant configuré pour :
- recevoir dudit dispositif de mesure (45) les tensions électriques du composant thermo-résistif (42) ;
- calculer les valeurs résistives (R) respectives indicatives de la résistance électrique du composant thermo-résistif (42).

11. Appareil de mesure selon l'une quelconque des revendications précédentes, ledit débitmètre (34) étant un microsystème électromécanique intégré de forme miniaturisée, généralement connu sous le nom de MEMS (microsystème électromécanique) ; en particulier, ledit débitmètre (34) étant miniaturisé.

12. Appareil de mesure selon l'une quelconque des revendications précédentes, l'élément de réglage (35) et le débitmètre (34) délimitant latéralement une même section de passage prédéfinie (Q) du canal (C3) ; l'élément de réglage (35) étant configuré pour obtenir un débit prédéfini du liquide (LU) qui s'écoule au contact du débitmètre (34).

13. Appareil de mesure selon l'une quelconque des revendications précédentes et comprenant un capteur optique (36) ; en particulier, ledit appareil de mesure comprend en outre un élément réfléchissant (37) qui fait face au capteur optique (36) de sorte que, lors de l'utilisation, le liquide (LU) s'écoule entre le capteur optique (36) et l'élément réfléchissant (37).

14. Dispositif de diurèse artificielle comprenant un appareil de mesure (S3) selon l'une quelconque des revendications précédentes.
